# EUROPÄISCHE PATENTSCHRIFT

(11) **EP 0 654 036 B1**
(45) Veröffentlichungstag und Bekanntmachung des Hinweises auf die Patenterteilung: **23.04.1997**
(21) Anmeldenummer: 93917764.8
(22) Anmeldetag: 06.08.1993
(51) Int. Cl.: C07F 9/6561, A61K 31/675

(54) **LIPONUCLEOTIDE VON SECO-NUCLEOSIDEN, DEREN HERSTELLUNG SOWIE DEREN VERWENDUNG ALS ANTIVIRALE ARZNEIMITTEL**
LIPONUCLEOTIDES OF SECO-NUCLEOSIDES, THEIR PREPARATION AND THEIR USE AS ANTI-VIRAL DRUGS
LIPONUCLEOTIDES DE SECO-NUCLEOSIDES, LEUR FABRICATION ET LEUR UTILISATION COMME MEDICAMENTS ANTIVIRAUX

(30) Priorität: 08.08.1992 DE 4226279
(43) Veröffentlichungstag der Anmeldung: 24.05.1995
(73) Patentinhaber: BOEHRINGER MANNHEIM GMBH, 68298 Mannheim (DE)
(72) Erfinder: ZILCH, Harald, D-68305 Mannheim (DE); HERRMANN, Dieter, D-69115 Heidelberg (DE)
(86) Internationale Anmeldenummer: EP9302101
(87) Internationale Veröffentlichungsnummer: WO9403465

(56) Entgegenhaltungen:
- EP-A- 0 350 287
- WO-A-92/03462
- ACTA CHEMICA SCANDINAVICA Bd. B39, Nr. 1, 1985, Copenhagen, DK, Seiten 47-54, C.J. WELCH

## Beschreibung

Gegenstand der vorliegenden Erfindung sind neue Phospholipid-Derivate von Seco-Nucleosiden, die einen Lipidteil, der ein substituiertes C₃-Grundgerüst darstellt, über ein Phosphat oder Thiophosphat mit einem Seco-Nucleosid verküpfen, sowie deren Verwendung als antivirale Arzneimittel.

Die Erfindung betrifft Verbindungen der Formel I, 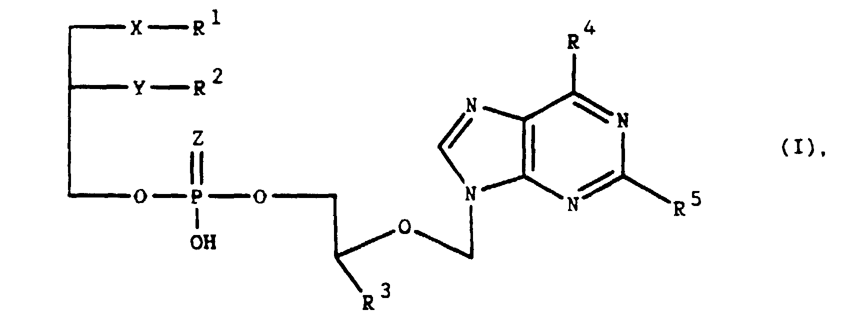 in der
R¹ ein geradkettiger oder verzweigter, gesättigter oder ungesättigter aliphatischer Rest mit 1-20 Kohlenstoffatomen bedeutet, der gegebenenfalls ein- oder mehrfach durch Phenyl-, Halogen, C₁-C₆-Alkoxy-, C₁-C₆-Alkylmercapto-, C₁-C₆-Alkoxycarbonyl-, C₁-C₆-Alkylsulfinyl- oder C₁-C₆-Alkylsulfonylgruppen substituiert sein kann,
R² ein geradkettiger oder verzweigter, gesättigter oder ungesättigter aliphatischer Rest mit 1-20 Kohlenstoffatomen bedeutet, der gegebenenfalls ein- oder mehrfach durch Phenyl-, Halogen-, C₁-C₆-Alkoxy-, C₁-C₆-Alkylmercapto-, C₁-C₆-Alkoxycarbonyl-, C₁-C₆-Alkylsulfinyl- oder C₁-C₆-Alkylsulfonylgruppen substituiert sein kann,
R³ Wasserstoff oder eine gegebenenfalls durch Hydroxy substituierte C₁-C₆-Alkylgruppe bedeutet,
R⁴ Wasserstoff, Hydroxy, Amino oder eine durch C₁-C₆-Alkyl ein- oder zweifach substituierte Aminogruppe sein kann,
R⁵ Wasserstoff, Hydroxy, Amino oder eine durch C₁-C₆-Alkyl ein- oder zweifach substituierte Aminogruppe sein kann,
X einen Valenzstrich; Sauerstoff, Schwefel, Sulfinyl oder Sulfonyl darstellt,
Y die gleiche Bedeutung von X hat, wobei die beiden Gruppen X und Y gleich oder verschieden sein können,
Z Sauerstoff oder Schwefel sein kann,
deren Tautomere und deren physiologisch verträgliche Salze anorganischer und organischer Säuren oder Basen.

Da die Verbindungen der allgemeinen Formel I asymmetrische Kohlenstoffatome enthalten, sind auch sämtliche optisch aktiven Formen und racemische Gemische dieser Verbindungen Gegenstand der vorliegenden Erfindung.

In J. Biol. Chem. 265, 6112 (1990) und EP 0 350 287 ist die Herstellung und Verwendung von Liponucleotiden als antivirale Arzneimittel beschrieben. Untersucht und synthetisiert wurden hier aber nur die an bekannte Nucleoside, wie z.B. AZT (Azidothymidin) und ddC (Didesoxycytidin), gekoppelten Dimyristoylphosphatidyl-und Dipalmitoylphosphatidylreste mit ihrer Fettsäureesterstruktur. EP 0 350 287 beschreibt die entsprechenden 1,2-Diester des Glycerins.

In J. Med. Chem. 33, 1380 (1990) sind Nucleosid-Konjugate von Thioetherlipiden mit Cytidindiphosphat beschrieben, die eine antitumorale Wirkung aufweisen und Verwendung in der Onkologie finden.

In Chem. Pharm. Bull. 36, 209 (1988) sind 5'-(3-SN-Phosphatidyl)nucleoside mit antileukämischer Aktivität beschrieben, sowie deren enzymatische Synthese aus den entsprechenden Nucleosiden und Phosphocholinen in Gegenwart von Phospholipase D mit Transferaseaktivität.

In der Patentanmeldung PCT/EP91/01541 sind Liponucleotide mit cyclischem Zuckerteil im Nukleosid mit antiviraler Wirkung beschrieben.

Das Acyclovir-Phospholipid-Konjugat aus L-α-Dimyristoylphosphatidylsäure und Acyclovir ist in Acta Chem. Scand., Ser. B, 39, 47 (1985) beschrieben [Vgl. auch Organophosphorus Chem. 18, 187 (1987)].

Die EP-A-350 287 beschreibt Etherlipid-phosphat-Nucleosid-Konjugate, darunter auch Acylglycerol-phosphat-AZT-Konjugate, als antiviral wirksame Verbindungen.

Die Ether-/Thioetherlipide (X, Y = O oder S) der vorliegenden Erfindung sind neu und weisen ebenfalls wertvolle pharmakologische Eigenschaften auf. Insbesondere eignen sie sich zur Therapie und Prophylaxe von Infektionen, die durch DNA-Viren wie z.B. das Herpes-Simplex-Virus, das Zytomegalie-Virus, Papilloma-Viren, das Varicella-Zoster-Virus oder Epstein-Barr-Virus oder RNA-Viren wie Toga-Viren oder Retroviren wie die Onko-Viren HTLV-I und II, sowie die Lentiviren Visna und Humanes-Immunschwäche-Virus HIV-1 und 2, verursacht werden.

Besonders geeignet erscheinen die Verbindungen der Formel I zur Behandlung der klinischen Manifestationen der viralen Herpes-Infektion beim Menschen. Die Verbindungen der allgemeinen Formel I wirken antiviral, ohne in pharmakologisch relevanten Dosen cytotoxisch zu sein.

Die Verbindungen zeichnen sich ferner durch eine sehr gute orale Verträglichkeit bei guter Bioverfügbarkeit aus.

Die Verbindungen der vorliegenden Erfindung und ihre pharmazeutischen Zubereitungen können auch in Kombination mit anderen Arzneimitteln zur Behandlung und Prophylaxe der oben genannten Infektionen eingesetzt werden. Beispiele dieser weiteren Arzneimittel beinhalten Mittel, die zur Behandlung und Prophylaxe von HIV-Infektionen oder diese Krankheit begleitende Erkrankungen einsetzbar sind wie 3'-Azido-3'-desoxythymidin (AZT), 2',3'-Didesoxynucleoside wie z. B. 2',3'-Didesoxycytidin (ddC), 2',3'-Didesoxyadenosin und 2',3'-Didesoxyinosin (ddI) oder nicht-nucleosidische RT-Inhibitoren, wie z. B. HEPT, Nevirapin oder L-697,661 und entsprechende Derivate. Die Verbindungen der vorliegenden Erfindung und das andere Arzneimittel können jeweils einzeln, gleichzeitig gegebenenfalls in einer einzigen oder zwei getrennten Formulierungen oder zu unterschiedlichen Zeiten verabreicht werden.

Als mögliche Salze der Verbindungen der allgemeinen Formel I kommen vor allem Alkali-, Erdalkali- und Ammoniumsalze der Phosphatgruppe in Frage. Als Alkalisalze sind Lithium-, Natrium- und Kaliumsalze bevorzugt. Als Erdalkalisalze kommen insbesondere Magnesium- und Calciumsalze in Frage. Unter Ammoniumsalzen werden erfindungsgemäß Salze verstanden, die das Ammoniumion enthalten, das bis zu vierfach durch Alkylreste mit 1-4 Kohlenstoffatomen und/oder Aralkylreste, bevorzugt Benzylreste, substituiert sein kann. Die Substituenten können hierbei gleich oder verschieden sein.

Die Verbindungen der allgemeinen Formel I können basische Gruppen, insbesondere Amino-Gruppen enthalten, die mit geeigneten organischen oder anorganischen Säuren in Säureadditionssalze überführt werden können. Als Säuren kommen hierfür beispielsweise in Betracht: Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure.

In der allgemeinen Formel I bedeutet R¹ vorzugsweise eine geradkettige C₉-C₁₄-Alkylgruppe, die noch durch eine C₁-C₆-Alkoxy oder eine C₁-C₆-Alkylmercaptogruppe substituiert sein kann. R¹ stellt insbesondere eine Decyl-, Undecyl-, Dodecyl-, Tridecyl- oder Tetradecylgruppe dar. Als C₁-C₆-Alkoxysubstituenten von R¹ kommen vorzugsweise die Methoxy-, Ethoxy-, Butoxy- und die Hexyloxygruppen in Frage. Ist R¹ durch einen C₁-C₆-Alkylmercaptorest substituiert, versteht man darunter insbesondere den Methylmercapto-, Ethylmercapto-, Propylmercapto-, Butylmercapto- und den Hexylmercaptorest.

R² bedeutet vorzugsweise eine geradkettige C₉-C₁₄-Alkylgruppe, die noch durch eine C₁-C₆-Alkoxygruppe oder eine C₁-C₆-Alkylmercaptogruppe substituiert sein kann. R² stellt insbesondere eine Decyl-, Undecyl-, Dodecyl-, Tridecyl- oder Tetradecylgruppe dar. Als C₁-C₆-Alkoxysubstituenten von R² kommen vorzugsweise die Methoxy-, Ethoxy-, Propoxy-, Butoxy- und die Hexyloxygruppe in Frage. Ist R² durch einen C₁-C₆-Alkylmercaptorest substituiert, versteht man darunter insbesondere den Methylmercapto-, Ethylmercapto-, Butylmercapto- und Hexylmercaptorest.

In der Definition von R³ bedeutet die Alkylgruppe insbesondere eine geradkettige oder verzweigte Alkylgruppe mit vorzugsweise bis zu vier C-Atomen, wie z. B. Methyl, Ethyl, n-Propyl-, iso-Propyl oder n-Butyl. Diese Alkylgruppen sind vorzugsweise durch eine oder zwei Hydroxygruppen substituiert, wie z. B. Hydroxymethyl-, 2-Hydroxyethyl oder 3-Hydroxypropyl.

C₁-C₆-Alkylgruppen bedeuten generell geradkettige oder verzweigte Alkylreste mit vorzugsweise bis zu vier C-Atomen, wie z. B. Methyl, Ethyl, n-Propyl, iso-Propyl, n-Butyl oder iso-Butyl.
- R⁴: bedeutet vorzugsweise eine Hydroxy- oder Aminogruppe.
- R⁵: bedeutet insbesondere Wasserstoff oder eine Hydroxy- oder Aminogruppe.

X und Y stellen bevorzugt ein Sauerstoff- oder Schwefelatom dar. Z ist bevorzugt ein Sauerstoffatom.

Besonders bevorzugte gekoppelte Seco-Nucleoside in den beanspruchten Liponucleotiden der allgemeinen Formel I sind Ganciclovir oder Acyclovir.

Die Verbindungen der Formel I können dargestellt werden, in dem man
1. eine Verbindung der Formel II, 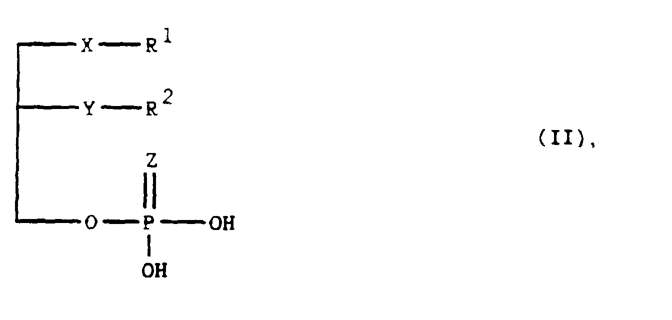 in der R¹, R², X, Y und Z die angegebenen Bedeutungen besitzen, mit einer Verbindung der allgemeinen Formel III, 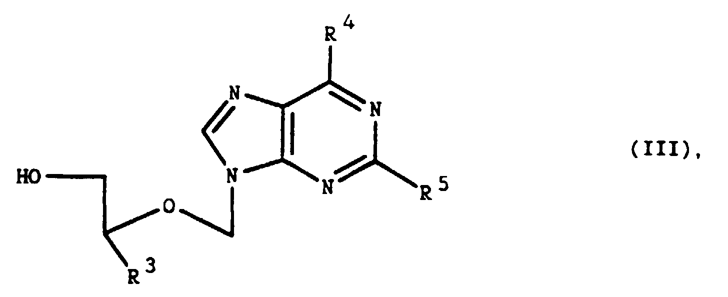 in der R³, R⁴ und R⁵ die oben angegebene Bedeutung besitzen, mit einem Kondensierungsmittel wie DCC (Dicyclohexylcarbodiimid) in Pyridin oder in Gegenwart von 2,4,6-Triisopropylbenzolsulfonsäurechlorid und einer tert. Stickstoffbase, z. B. Pyridin oder Lutidin, in einem inerten Lösungsmittel, wie z. B. Toluol, oder direkt in Pyridin zur Reaktion bringt und nach erfolgter Hydrolyse gegebenenfalls entsprechend den in der Nucleosidchemie üblichen Verfahren die Sauerstoffschutzgruppen abspaltet, oder
2. eine Verbindung der Formel IV 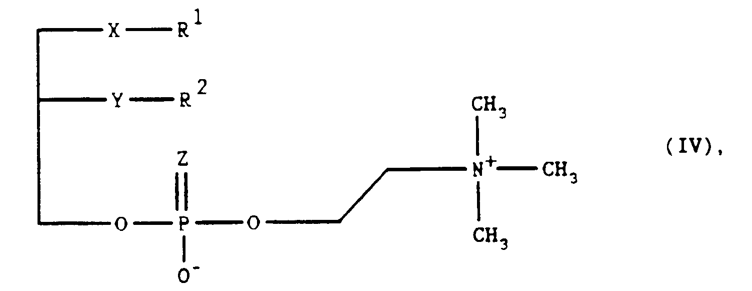 in der R¹, R², X, Y und Z die oben genannten Bedeutungen besitzen, mit einer Verbindung der Formel III, in der R³, R⁴ und R⁵ die angegebenen Bedeutungen besitzen, in Gegenwart von Phospholipase D in einem inerten Lösungsmittel, wie z. B. Chloroform, in Gegenwart eines geeigneten Puffers zur Reaktion bringt und nach erfolgter Reaktion gegebenenfalls entsprechend den in der Nucleosidchemie üblichen Verfahren die Sauerstoffschutzgruppe abspaltet.

Die Herstellung der Verbindungen der Formel II und IV sind in DE 39 29 217.7 bzw. WO 91/05558 beschrieben.

Die Herstellung der Verbindungen der allgemeinen Formel III ist in Progress in Medicinal Chemistry, Vol. 23, 187 (1986) und der dort zitierten Literatur beschrieben.

Acyclovir und Ganciclovir sind käuflich zu erwerben.

Die Arzneimittel enthaltend Verbindungen der Formel I zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragees, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage:
1. 2'-[9-(Ethoxymethyl)guanin]phosphorsäure-(3-dodecylmercapto-2-decyloxy)-1-propylester
2. 2'-(9-{[(1-Hydroxymethyl)ethoxy]methyl}guanin)phosphorsäure-(3-dodecylsulfinyl-2-decyloxy)-1-propylester
3. 2'-(9-{[(1-Hydroxymethyl)ethoxy]methyl}guanin)phosphorsäure-(3-dodecylsulfonyl-2-decyloxy)-1-propylester
4. 2'-(9-{[(1-Hydroxymethyl)ethoxy]methyl}guanin)phosphorsäure-(3-dodecylmercapto-2-decyloxy)-1-propylester
5. 2'-[9-(Ethoxymethyl)guanin]phosphorsäure-(3-undecylmercapto-2-decyloxy)-1-propylester
6. 2'-[9-(Ethoxymethyl)guanin]phosphorsäure-(3-dodecyloxy-2-decyloxy)-1-propylester
7. 2'-(9-{[(1-Hydroxymethyl)ethoxy]methyl}guanin)phosphorsäure-(3-dodecylmercapto-2-nonyloxy)-1-propylester
8. 2'-(9-{[(1-Hydroxymethyl)ethoxy]methyl}quanin)phosphorsäure-(3-dodecylmercapto-2-decylmercapto)-1-propylester
9. 2'-(9-{[(1-Hydroxymethyl)ethoxy]methyl}guanin)phosphorsäure-(3-undecylmercapto-2-decyloxy)-1-propylester
10. 2'-[9-(Ethoxymethyl)guanin]phosphorsäure-(3-tridecylmercapto-2-decyloxy)-1-propylester
11. 2'-(9-{[(1-Hydroxymethyl)ethoxy]methyl}guanin)phosphorsäure-(3-tridecylmercapto-2-decyloxy)-1-propylester
12. 2'-[9-(Ethoxymethyl)guanin]phosphorsäure-(3-dodecylmercapto-2-dodecyloxy)-1-propylester
13. 2'-[9-(Ethoxymethyl)guanin]phosphorsäure-(3-dodecylmercapto-2-undecyloxy)-1-propylester
14. 2'-(9-{[(1-Hydroxymethyl)ethoxy]methyl}guanin)phosphorsäure-(2, 3-bis(dodecylmercapto)-1-propylester
15. 2'-(9-{[(1-Hydroxymethyl)ethoxy]methyl}guanin)phosphorsäure-(3-decylmercapto-2-dodecyloxy)-1-propylester
16. 2'-[9-(Ethoxymethyl)guanin]phosphorsäure-(3-undecyloxy-2-dodecyloxy)-1-propylester
17. 2'-[9-(Ethoxymethyl)guanin]phosphorsäure-(3-decylsulfonyl-2-dodecyloxy)-1-propylester
18. 2'-[9-(Ethoxymethyl)guanin]phosphorsäure-(3-decyloxy-2-decyloxy)-1-propylester
19. 2'-[9-(Ethoxymethyl)guanin]phosphorsäure-(3-dodecylmercapto-2-dodecyloxy)-1-propylester
20. 2'-(9-{[(1-Hydroxymethyl)ethoxy]methyl}quanin)phosphorsäure-(3-tetradecylmercapto-2-decyloxy)-1-propylester
21. 2'-[9-(Ethoxymethyl)guanin]phosphorsäure-(3-pentadecylmercapto-2-decyloxy)-1-propylester
22. 2'-(9-{[(1-Hydroxymethyl)ethoxy]methyl}guanin)phosphorsäure-(3-tridecylmercapto-2-decyloxy)-1-propylester
23. 2'-[9-(Ethoxymethyl)guanin]phosphorsäure-(3-dodecylmercapto-2-octyloxy)-1-propylester

### Beispiel 1

### Phosphorsäure-(3-dodecylmercapto-2-decyloxy)-1-propylester

Eine Suspension aus 4.26 g P₄O₁₀ in 60 ml abs. Pyridin wurde bei RT mit 13 ml Hexamethyldisiloxan versetzt und 1 h auf 100°C erhitzt. Dann wurde leicht abgekühlt, mit 25 g 3-Dodecylmercapto-2-decyloxy-1-propanol versetzt und weitere 2.5 h auf 100°C erwärmt.

Nach dem vollständigen Abkühlen auf RT und Entfernung der leicht flüchtigen Bestandteile im Vakuum, konnte das Phosphat mit Ether aus einer wässrigen Suspension des Rückstandes extrahiert werden. Der Eindampfrückstand der Etherphase wurde durch Säulenchromatographie an Kieselgel 60 bzw. RP 18 gereinigt. Ausbeute 18.7 g (63 %), R_{f} = 0.66 (CH₂Cl₂/MeOH/H₂O 6.5/2.5/0.4) auf DC-Platten Merck 5715, Kieselgel 60.

### Beispiel 2

### 2'-(9-{[(1-Hydroxymethyl)ethoxy]methyl}guanin)phosphorsäure-(3-dodecyl mercapto-2-decyloxy)-1-propylester

1.45 g (3 mmol) Phosphorsäure-(3-dodecylmercapto-2-decyloxy)-1-propylester und 770 mg (3 mmol) Ganciclovir wurden zweimal mit je 20 ml abs. Pyridin versetzt und eingedampft. Der Rückstand wurde in 20 ml abs. Pyridin aufgenommen, unter Stickstoff mit 2.7 g (8.5 mmol) 2,4,6-Triisopropylbenzolsulfonsäurechlorid versetzt und 24 h bei 40°C gerührt. Dann wurden 10 ml Wasser zugegeben, die Mischung weitere 2 h bei Raumtemperatur gerührt und das Lösungsmittel im Rotationsverdampfer entfernt.

Der ölige Rückstand wurde durch Abdampfen mit Toluol von Pyridinresten befreit und durch Säulenchromatographie an RP 18 mit einem linearen Gradienten von Methanol/Wasser 7/3 bis 9.5/0.5 als Eluens gereinigt. Ausbeute 0.75 g (34 % d.Th.), Öl. R_{f} = 0.73 (H₂O/MeOH 0.5/9.5) auf RP 8, R_{f} = 0.30 (CH₂Cl₂/MeOH/H₂O 6.5/2.5/0.4) auf DC-Platten Merck 5715 Kieselgel 60 F.

### Beispiel 3

### 2'-[9-(Ethoxymethyl)guanin]phosphorsäure-(3-dodecylmercapto-2-decyloxy)-1-propylester

Diese Verbindung wurde wurde analog zu Bsp. 1 aus Acyclovir in 47 % Ausbeute hergestellt, Öl, R_{F} = 0.77 (H₂O/MeOH 0.5/9.5) auf RP 8, R_{f} = 0.35 (CH₂Cl₂/MeOH/H₂O 6.5/2.5/0.4) auf DC-Platten Merck 5715, Kieselgel 60.

## Patentansprüche

1. Liponucleotide der Formel I, 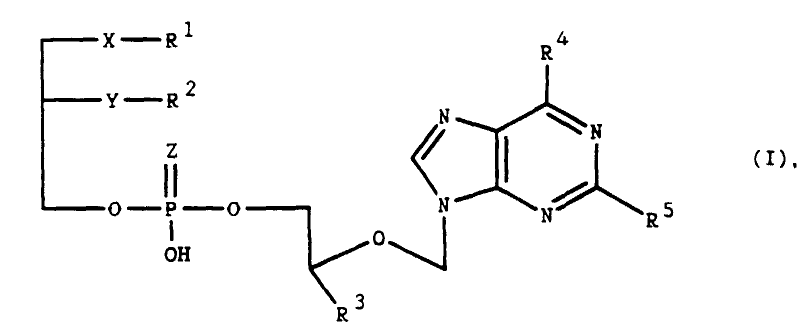 in der
R¹ eine geradkettige oder verzweigte, gesättigte oder ungesättigte Alkylkette mit 1-20 Kohlenstoffatomen, die gegebenenfalls ein oder mehrfach durch Phenyl-, Halogen, C₁-C₆-Alkoxy-, C₁-C₆-Alkylmercapto-, C₁-C₆-Alkoxycarbonyl-, C₁-C₆-Alkylsulfinyl- oder C₁-C₆-Alkylsulfonylgruppen substituiert sein kann,
R² eine geradkettige oder verzweigte, gesättigte oder ungesättigte Alkylkette mit 1-20 Kohlenstoffatomen, die gegebenenfalls ein oder mehrfach durch Phenyl-, Halogen-, C₁-C₆-Alkoxy-, C1-C6-Alkylmercapto-, C₁-C₆-Alkoxycarbonyl-, C₁-C₆-Alkylsulfinyl- oder C₁-C₆-Alkylsulfonylgruppen substituiert sein kann,
R³ Wasserstoff oder eine gegebenenfalls durch Hydroxy substituierte C₁-C₆-Alkylgruppe bedeutet,
R⁴ Wasserstoff, Hydroxy, Amino oder eine durch C₁-C₆-Alkyl ein- oder zweifach substituierte Aminogruppe sein kann,
R⁵ Wasserstoff, Hydroxy, Amino oder eine durch C₁-C₆-Alkyl ein- oder zweifach substituierte Aminogruppe sein kann,
X einen Valenzstrich, Sauerstoff, Schwefel, Sulfinyl oder Sulfonyl darstellt,
Y die gleiche Bedeutung von X hat, wobei die beiden Gruppen X und Y gleich oder verschieden sein können,
Z Sauerstoff oder Schwefel sein kann,
deren Tautomere und deren physiologisch verträgliche Salze anorganischer und organischer Säuren oder Basen.

2. Liponukleotide gemäß Anspruch 1, dadurch gekennzeichnet, daß R¹ eine geradkettige C₉-C₁₄-Alkylgruppe bedeutet, die durch eine C₁-C₆-Alkoxy oder eine C₁-C₆-Alkylmercaptogruppe substituiert sein kann.

3. Liponukleotide gemäß Anspruch 2, dadurch gekennzeichnet, daß R¹ eine Decyl-, Undecyl-, Dodecyl-, Tridecyl- oder Tetradecylgruppe bedeutet, die durch eine Methoxy-, Ethoxy-, Butoxy-, Hexyloxy-, Methylmercapto-, Ethylmercapto-, Propylmercapto-, Butylmercapto- oder Hexylmercaptogruppe substituiert sein kann.

4. Liponukleotide gemäß einem der Ansprüche 1-3, dadurch gekennzeichnet, daß R² eine geradkettige C₉-C₁₄-Alkylgruppe bedeutet, die durch eine C₁-C₆-Alkoxy- oder C₁-C₆-Alkylmercaptogruppe substituiert sein kann.

5. Liponukleotide gemäß Anspruch 4, dadurch gekennzeichnet, daß R² eine Decyl-, Undecyl-, Dodecyl-, Tridecyl- oder Tetradecylgruppe bedeutet, die durch eine Methoxy-, Ethoxy-, Propoxy-, Butoxy-, Hexyloxy-, Methylmercapto-, Ethylmercapto-, Butylmercapto- oder Hexylmercaptogruppe substituiert sein kann.

6. Liponukleotide gemäß einem der Ansprüche 1-5, dadurch gekennzeichnet, daß R³ Wasserstoff oder Hydroxy C₁-C₆-alkyl bedeutet.

7. Liponukleotide gemäß einem der Ansprüche 1-6, dadurch gekennzeichnet, daß R⁴ Hydroxy oder Amino bedeutet.

8. Liponukleotide gemäß einem der Ansprüche 1-7, dadurch gekennzeichnet, daß R⁵ Wasserstoff oder Amino bedeutet.

9. Verfahren zur Herstellung von Liponukleotiden der Formel I gemäß einem der Ansprüche 1-8, dadurch gekennzeichnet, daß man
a) eine Verbindung der Formel II, 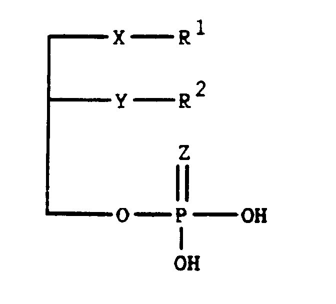 in der R¹, R², X, Y und Z die angegebenen Bedeutungen besitzen, mit einer Verbindung der allgemeinen Formel III, 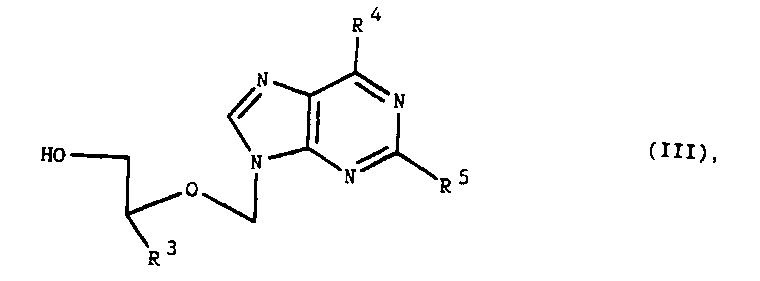 in der R³, R⁴ und R⁵ die oben angegebene Bedeutung besitzen, mit einem Kondensierungsmittel in einem inerten Lösungsmittel zur Reaktion bringt und nach erfolgter Hydrolyse gegebenenfalls entsprechend den in der Nucleosidchemie üblichen Verfahren die eventuell vorhandenen Sauerstoffschutzgruppen abspaltet, oder
b) eine Verbindung der Formel IV 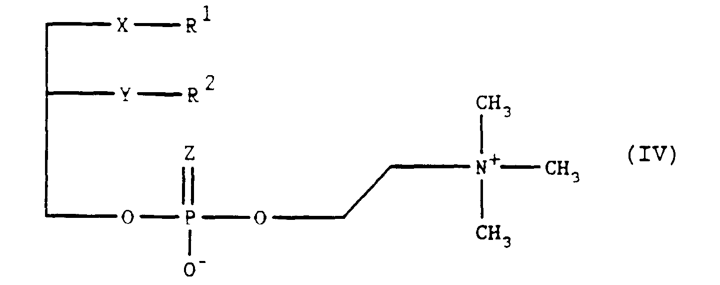 in der R¹, R², X, Y und Z die oben genannten Bedeutungen besitzen, mit einer Verbindung der Formel III, in der R³, R⁴ und R⁵ die angegebenen Bedeutungen besitzen, in Gegenwart von Phospholipase D in einem inerten Lösungsmittel in Gegenwart eines geeigneten Puffers zur Reaktion bringt und nach erfolgter Reaktion gegebenenfalls entsprechend den in der Nucleosidchemie üblichen Verfahren eventuell vorhandene Sauerstoffschutzgruppe abspaltet, und
anschließend gegebenenfalls die Verbindungen der Formel I in ihre physiologisch verträgliche Salze überführt.

10. Arzneimittel enthaltend mindestens ein Liponucleotid der Formel I gemäß einem der Ansprüche 1-8 sowie weitere pharmazeutisch übliche Hilfs- oder Trägerstoffe.

11. Verwendung von Liponukleotiden der Formel I gemäß einem der Ansprüche 1-8 zur Herstellung von Arzneimitteln zur Behandlung von viralen oder retroviralen Infektionen.

## Claims

1. Liponucleotides of formula I 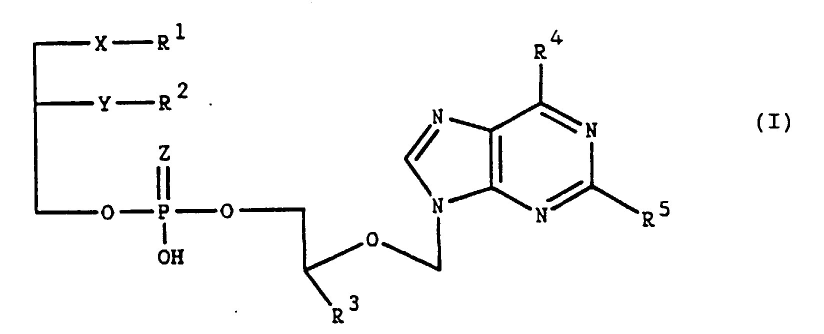 in which
R¹ denotes a straight-chained or branched, saturated or unsaturated alkyl chain with 1-20 carbon atoms which can be substituted if desired once or several times by phenyl, halogen, C₁-C₆ alkoxy, C₁-C₆ alkylmercapto, C₁-C₆ alkoxycarbonyl, C₁-C₆ alkylsulfinyl or C₁-C₆ alkylsulfonyl groups,
R² denotes a straight-chained or branched, saturated or unsaturated alkyl chain with 1-20 carbon atoms which can be substituted if desired once or several times by phenyl, halogen, C₁-C₆ alkoxy, C₁-C₆ alkylmercapto, C₁-C₆ alkoxycarbonyl, C₁-C₆ alkylsulfinyl or C₁-C₆ alkylsulfonyl groups,
R³ denotes hydrogen or a C₁-C₆ alkyl group which is substituted if desired by hydroxy,
R⁴ can be hydrogen, hydroxy, amino or an amino group substituted once or twice by C₁-C₆ alkyl,
R⁵ can be hydrogen, hydroxy, amino or an amino group substituted once or twice by C₁-C₆ alkyl,
X represents a valency dash, oxygen, sulphur, sulfinyl or sulfonyl,
Y has the same meaning as X and the two groups X and Y can be the same or different,
Z can be oxygen or sulphur,
their tautomers and their physiologically tolerated salts of inorganic and organic acids and bases.

2. Liponucleotides as claimed in claim 1, wherein R¹ denotes a straight-chained C₉-C₁₄ alkyl group which can be substituted by a C₁-C₆ alkoxy or a C₁-C₆ alkylmercapto group.

3. Liponucleotides as claimed in claim 2, wherein R¹ denotes a decyl, undecyl, dodecyl, tridecyl or tetradecyl group which can be substituted by a methoxy, ethoxy, butoxy, hexyloxy, methylmercapto, ethylmercapto, propylmercapto, butylmercapto or hexylmercapto group.

4. Liponucleotides as claimed in one of the claims 1-3, wherein R² denotes a straight-chained C₉-C₁₄ alkyl group which can be substituted by a C₁-C₆ alkoxy or C₁-C₆ alkylmercapto group.

5. Liponucleotides as claimed in claim 4, wherein R² denotes a decyl, undecyl, dodecyl, tridecyl or tetradecyl group which can be substituted by a methoxy, ethoxy, propoxy, butoxy, hexyloxy, methylmercapto, ethylmercapto, butylmercapto or hexylmercapto group.

6. Liponucleotides as claimed in one of the claims 1-5, wherein R³ denotes hydrogen or hydroxy C₁-C₆ alkyl.

7. Liponucleotides as claimed in one of the claims 1-6, wherein R⁴ denotes hydroxy or amino.

8. Liponucleotides as claimed in one of the claims 1-7, wherein R⁵ denotes hydrogen or amino.

9. Process for the production of liponucleotides of formula I as claimed in one of the claims 1-8, wherein
a) a compound of formula II, 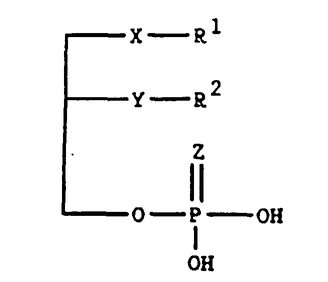 in which R¹, R², X, Y and Z have the stated, meanings is reacted in an inert solvent with a compound of the general formula III, 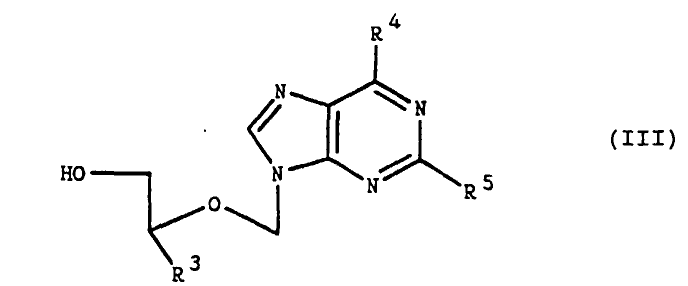 in which R³, R⁴ and R⁵ have the above-mentioned meaning, using a condensing agent and after completion of hydrolysis the oxygen protecting groups which may be present are cleaved off if desired according to conventional methods in nucleoside chemistry or
b. a compound of formula IV 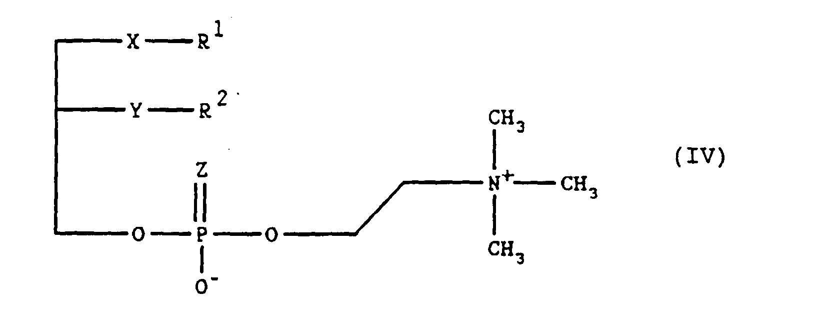 in which R¹, R², X, Y and Z have the above-mentioned meanings is reacted with a compound of formula III, in which R³, R⁴ and R⁵ have the stated meanings, in the presence of phospholipase D in an inert solvent in the presence of a suitable buffer and after the reaction is completed the oxygen protecting group which may be present is cleaved off if desired according to conventional methods in nucleoside chemistry, and
subsequently compounds of formula I are converted into their physiologically tolerated salts if desired.

10. Pharmaceutical agent containing at least one liponucleotide of formula I as claimed in one of the claims 1-8 as well as further conventional pharmaceutical auxiliary or carrier substances.

11. Use of liponucleotides of formula I as claimed in one of the claims 1-8 for the production of pharmaceutical agents for the treatment of viral or retroviral infections.

## Revendications

1. Liponucléotides de formule I. 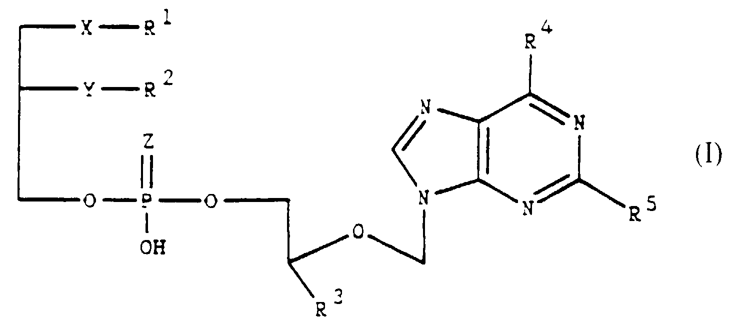 dans laquelle
R¹ représente une chaîne alkyle linéaire ou ramifiée, saturée ou insaturée, comportant 1 à 20 atomes de carbone, éventuellement substituée une ou plusieurs fois par un groupe phényle, halogéno, alcoxy en C₁-C₆, alkylmercapto en C₁-C₆, alcoxycarbonyle en C₁-C₆, alkylsulfinyle en C₁-C₆ ou alkylsulfonyle en C₁-C₆,
R² représente une chaîne alkyle linéaire ou ramifiée, saturée ou insaturée, comportant de 1 à 20 atomes de carbone, éventuellement substituée une ou plusieurs fois par un groupe phényle, halogéno, alcoxy en C₁-C₆, alkylmercapto en C₁-C₆, alcoxycarbonyle en C₁-C₆, akylsulfinyle en C₁-C₆ ou alkylsulfonyle en C₁-C₆,
R³ représente un atome d'hydrogène ou un groupe alkyle en C₁-C₆ éventuellement substitué par un groupe hydroxy,
R⁴ représente un atome d'hydrogène, un groupe hydroxy, amino ou un groupe amino portant un ou deux substituants alkyle en C₁-C₆,
R⁵ représente un atome d'hydrogène, un groupe hydroxy, amino ou un groupe amino portant un ou deux substituants alkyle en C₁-C₆,
X représente une valence libre, un atome d'oxygène, un atome de soufre, un groupe sulfinyle ou un groupe sulfonyle,
Y a la même signification que X, les groupes X et Y pouvant être identiques ou différents,
Z représente un atome d'oxygène ou de soufre,
leurs tautomères et leurs sels physiologiquement acceptables d'acides ou de bases inorganiques ou organiques.

2. Liponucléotides selon la revendication 1, caractérisés en ce que R¹ représente un groupe alkyle à châIne linéaire en C₉-C₁₄ pouvant être substitué par un groupe alcoxy en C₁-C₆ ou alkylmercapto en C₁-C₆.

3. Liponucléotides selon la revendication 2, caractérisés en ce que R¹ représente un groupe décyle, undécyle, dodécyle, tridécyle ou tétradécyle pouvant être substitué par un groupe méthoxy, éthoxy, butoxy, hexyloxy, méthylmercapto, éthylmercapto, propylmercapto, butylmercapto ou hexylmercapto.

4. Liponucléotides selon l'une quelconque des revendications à 3, caractérisés en ce que R² représente un groupe alkyle à chaîne linéaire en C₉-C₁₄ pouvant être substitué par un groupe alcoxy en C₁-C₆ ou alkylmercapto en C₁-C₆.

5. Liponucléotides selon la revendication 4, caractérisés en ce que R² représente un groupe décyle, undécyle, dodécyle, tridécyle ou tétradécyle pouvant être substitué par un groupe méthoxy, éthoxy, propoxy, butoxy, hexyloxy, méthylmercapto, éthylmercapto, butylmercapto ou hexylmercapto.

6. Liponucléotides selon l'une quelconque des revendications 1 à 5, caractérisés en ce que R³ représente un atome d'hydrogène ou un groupe hydroxy(alkyle en C₁-C₆).

7. Liponucléotides selon l'une quelconque des revendications 1 à 6, caractérisés en ce que R⁴ est un groupe hydroxy ou amino.

8. Liponucléotides selon l'une quelconque des revendications 1 à 7, caractérisés en ce que R⁵ représente un atome d'hydrogène ou un groupe amino.

9. Procédé de préparation de liponucléotides de formule I selon l'une quelconque des revendications 1 à 8, caractérisé en ce que
a) on fait réagir un composé de formule II, 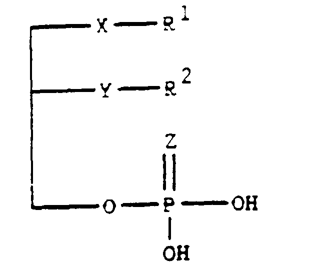 dans laquelle R¹, R², X, Y et Z ont les significations mentionnées, avec un composé de formule générale III, 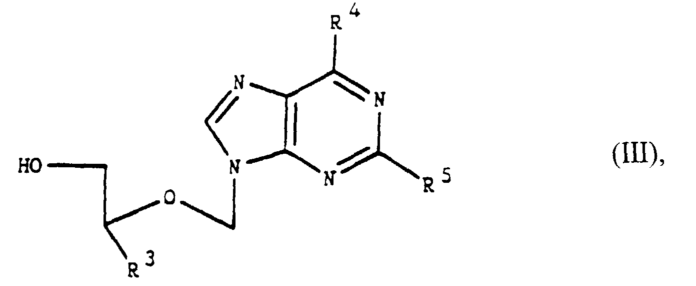 dans laquelle R³, R⁴ et R⁵ ont les significations mentionnées ci-dessus, avec un agent de condensation dans un solvant inerte et, une fois l'hydrolyse effectuée, on coupe le cas échéant les groupes de protection d'oxygène éventuellement présents, d'après le procédé usuel de la chimie des nucléosides, ou
b) on fait réagir un composé de formule IV 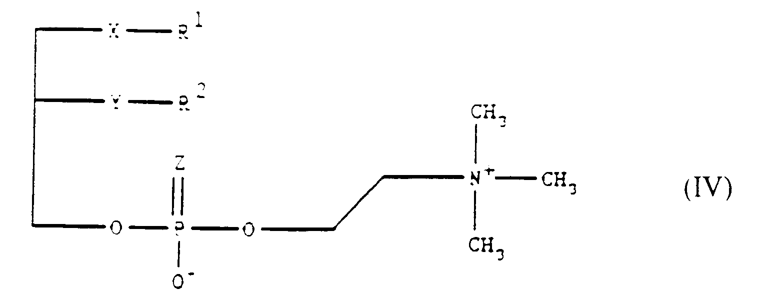 dans laquelle R¹, R², X, Y et Z ont les significations mentionnées ci-dessus, avec un composé de formule III, dans laquelle R³, R⁴ et R⁵ ont les significations mentionnées, en présence de phospholipase D dans un solvant inerte, en présence d'un tampon approprié et, une fois la réaction effectuée, on coupe le cas échéant le groupe de protection d'oxygène éventuellement présent d'après le procédé usuel de la chimie des nucléosides, puis,
on transforme éventuellement les composés de formule en leurs sels physiologiquement acceptables.

10. Médicament comprenant au moins un liponucléotide de formule I selon l'une quelconque des revendications 1 à 8 ainsi que d'autres adjuvants ou véhicules pharmaceutiques usuels.

11. Utilisation de liponucléotides de formule I selon l'une quelconque des revendications 1 à 8 pour la préparation de médicaments destinés au traitement d'infections virales ou rétrovirales.
